# EUROPEAN PATENT SPECIFICATION

(11) **EP 0 381 669 B1**
(45) Date of publication and mention of the grant of the patent: **11.10.1995**
(21) Application number: 88903630.7
(22) Date of filing: 11.03.1988
(51) Int. Cl.: G01N 33/53, C12Q 1/06

(54) **METHOD FOR SCREENING CELLS OR FORMED BODIES FOR ENUMERATION OF POPULATIONS EXPRESSING SELECTED CHARACTERISTICS**
METHODE ZUM SCREENING VON ZELLEN ODER ANDEREN GEFORMTEN KÖRPERN ZUR SPEZIFIZIERUNG VON POPULATIONEN, DIE AUSGEWÄHLTE CHARAKTERISTISCHE EIGENSCHAFTEN EXPRIMIEREN
PROCEDE DE SELECTION DE CELLULES DE CORPS FORMES POUR ENUMERATION DE POPULATIONS EXPRIMANT DES CARACTERISTIQUES SELECTIONNEES

(30) Priority: 13.03.1987 US 25345
(43) Date of publication of application: 16.08.1990
(73) Proprietor: COULTER CORPORATION, Hialeah, FL 33010 (US)
(72) Inventor: KORTRIGHT, Kenneth, H., Cooper City, FL 33328 (US); COULTER, Wallace, H., Miami Springs, FL 33166 (US); RODRIGUEZ, Carlos, Miami, FL 33175 (US); RUSSELL, Thomas, Miami, FL 33186 (US); PAUL, Ronald, North Miami Beach, FL 33184 (US)
(74) Representative: Perry, Robert Edward
(86) International application number: US8800960
(87) International publication number: WO8807199

(56) References cited:
- WO-A-84/02777
- GB-A- 2 173 004
- US-A- 3 970 518
- US-A- 4 284 412
- US-A- 4 499 052
- US-A- 4 599 307
- THE JOURNAL OF IMMUNOLOGY, vol. 136, no. 9, 1st May 1986, pages 3427-3432, The American Association of Immunologists, US; P.A.T. TETTEROO et al.: "Neutrophil activation detected by monoclonal antibodies", p. 3428

## Description

This invention relates generally to methods of using an automated analyzer for screening biological cells or formed bodies for the enumeration of populations which express selected characteristics for research, diagnostic, medical or industrial purposes. More particularly, the methods embodying the invention enable multiple part classifications of cells and formed bodies, functional phenotyping of cells and formed bodies, typing of leukemic, lymphoma and solid tumor cells, among others, using a unique combination of electronic technology and the specificity of selective biological molecules, such as antibodies, for such screening and selective enumeration of the cells and formed bodies.

### Background Art

Automation of routine complete blood cell (CBC) analysis of human peripheral blood by an automated blood cell counter was successfully achieved by the COULTER COUNTER® Model A of Coulter Electronics, Inc. of Hialeah, Florida. The electronic particle sensing system principle of that instrument is disclosed in U.S. Patent No. 2,656,508 issued October 20, 1953 to Wallace H. Coulter. The use of optical sensing means or lasers, which can be troublesome and expensive, are avoided by particle analyzing instrumentation solely operated on this Coulter electronic sensing principle.

This Coulter sensing principle was developed and expanded into more sophisticated instrumentation such as the COULTER COUNTER® Model S types of instruments which enabled CBC parameters, absolute cell counts, platelet count and morphology, red blood cell (RBC) morphology, interpretation of normal and abnormal blood specimens by special computer programs.

The Coulter electronic particle sensing principle employs an aperture sensing circuit using a direct current (DC) aperture supply. Such particle sensors are simple in structure, extremely rugged and reliable as attested to by the substantially universal acceptance of the COULTER COUNTER® automated analyzer in clinical laboratories in the United States and throughout the rest of the World. An improvement in this basic aperture sensing circuit was disclosed in U.S. Patent No. 3,502,974 issued in 1970 to Wallace Coulter and Walter Hogg. In addition to the standard direct current aperture supply, a high frequency aperture current was applied which enabled the sensing of an additional parameter for classification purposes. The high frequency aperture current produced a signal which is the function of the blood cell's internal conductivity as well as its volume. The signal produced simultaneously by the direct current aperture circuit is a conventional DC amplitude signal which provides an indication primarily of cell volume. The radio frequency amplitude is divided by the direct current pulse amplitude employing a high speed divider circuit to obtain a quotient which is a function of cell volume and internal resistance, conveniently referred to as "opacity". This principle is further described in U.S. Patent No. 3,502,973 also issued to Wallace Coulter and Walter Hogg, in 1970. This parameter has applicability in cell classification systems. Either a single or a pair of separate apertures could be utilized for this purpose.

Classification of different populations is accomplished by collating the data of the signal pairs as they are produced; one, a measure of particle volume and the other a measure of cell internal resistivity or opacity. A convenient form of presenting this data is by two-dimensional plots referred to as scatterplots or scattergrams. Such plots are well described in Flow Cytometry and Sorting, page 371; edited by Melamed Melaney, and Medelsohn, 1979, John Wiley & Sons, NY, NY.

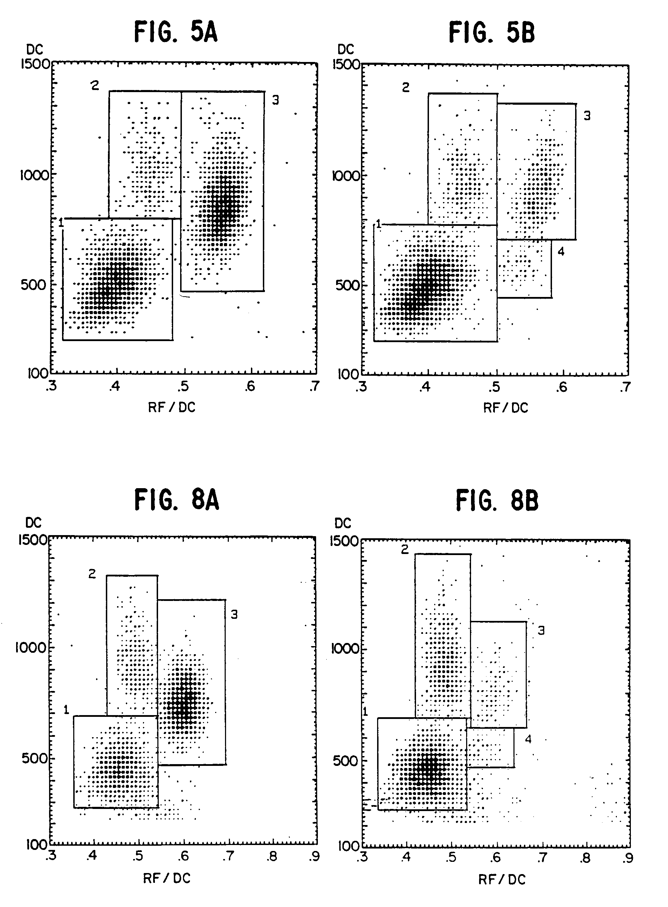

Fig. 5A is one example of a data plot of a sample of normal blood. Each dot represents an individual cell. The height above the baseline represents the relative volume of the cell. The distance of the dot to the right of the vertical baseline represents the relative opacity. A plot of normal white blood cells (WBC) (with the red blood cells removed) shows three clusters of dots representing three distinct populations which are a consequence of their intrinsic differences in size and internal composition. If desired, with suitable circuitry, these populations can be enumerated to obtain the numbers of each. The cells are classified on the basis of these inherent differences.

Initial applications of the Coulter electronic particle sensing principle was to perform red blood cell counts and then, more sophisticated determinations of other red blood cell parameters. By removing red blood cells from whole peripheral blood, analysis of the white blood cell populations could be undertaken so long as the red blood cell removal did not significantly impair properties of the remaining white blood cell populations sought to be measured. Red blood cell lysing reagents were developed for this purpose which, though useful and widely applied, were not entirely satisfactory in all respects for subsequent white blood cell determinations.

Previous methods of flow analysis of leukocytes using DC volume alone or light scatter at various angles have shown three clusters of leukocytes corresponding to lymphocytes, monocytes and granulocytes which included the neutrophil, basophil and eosinophil populations. A rough but useful estimation of eosinophil concentration can be made on some samples. The fifth major population is relatively too small for this approach. The eosinophils also have been observed as a distinct cluster using special fluorescence techniques.

These fluorescent techniques were utilised in flow cytometry instruments such as the EPICS® flow cytometer available from the Coulter Corporation. Such instruments employed the principle of cells moving ina columnar stream bounded by a sheath flow such that cells lined up in single file and passed individually through a laser beam. Light scatter and/or fluorescence signals from the cells were then utilised in classifying cell populations. Straining cells with absorptive or fluorescent dyes made additional cell population classifications possible. The development of instrumentation and fluorochromes for automated multiparameter analysis is further described by R.C.Leif, et al. in Clinical Chemistry, Vol.23, pp 1492-98 (1977). These developments expanded the number of simultaneous population classifications of leukocytes to four, namely lymphocytes, monocytes, eosinophils and "granulocytes" (neutrophils and basophils).

A more recent analytical hematology instrument has utilised light scattering techniques together with peroxidase enzyme staining (absorptive dye) of cells to produce a five part leukocyte differential. Moreover, dyes in combination with specific reacting biological molecules, such as monoclonal antibodies, have increased the number of leukocyte classifications possible to include functional sub-divisions.

GB-A-2173004 discloses apparatus for counting biological microparticles. In the apparatus, the microparticles are coupled to labelled antibodies to form complexes. A particle size measurement unit, of the scattered light or Coulter® type, is used to measure the size of particles in the blood including that of the complexes. When the measured size corresponds to that of a complex a processing unit generates an output signal and counts the number of these signals. The number of output signals corresponds to the number of complexes, and therefore to the number of microparticles bound within the complexes.

WO-A-8402777 discloses a system for differential determination of three populations of leukocytes. The system utilises, in combination, an osmotically-balanced blood diluent and a lysing reagent to modify the volume of at least one of the three populations of leukocytes, thereby allowing volumetric differential analysis. The data is presented using standard Coulter Counter® equipment in conjunction with a Coulter Channelyzer® and an X-Y plotter.

US-A-4499052 discloses a method of distinguishing multiple sub-populations of blood particles from a sample comprising a variety of particles, and apparatus for carrying out that method. Particles in the blood are labelled with at least two distinguishing, quantifiable marking agents in a plurality of different pre-selected ratios of those agents. The differentially-labelled particles are mixed with cells suspected of having specific receptors for them. Each cell is then analysed to determine the ratio of any two marking agents associated with the cell, and if this ratio is related to one of the pre-selected ratios of marking agents the cell can be classified in a sub-population category. Preferably antibody proteins are labelled with two fluorochromes having distinct emission spectra. Energy is provided to the cell to excite the fluorochromes, and the ratio of fluorescence emissions determined.

The invention herein provides a method which combines the application of electronic sensing aperture principles, the specificity of selective biological molecules for identifying and/or emunerating defined populations of cells or formed bodies and microscopic particle technology. An automated analyzer can be used together with a special lysing reagent and/or antibodies coupled to microscopic microspheres or supports of varying composition.

Selectively attaching microscopic particles makes possible the modification of the parameter(s) responsible for the original location of at least one of the populations. The bulk addition of microscopic particles to selected target populations where this addition affects the measured volume and/or opacity results in shifting the location of the dots representing a population.

Antibodies of known specificity are employed in coating microscopic particles. This coating gives the particle the capacity to selectively attach to certain cells which express the antigen the antibody is specific for. These coated or tagged cells are a combination of particles and cells which behave like a new entity. Their parameters of opacity, volume, or both opacity and volume may be considered to represent the sum of the effects of both the cell and the particles on the signals obtained. If the characteristics of the components are different, the new entity will move to a new position in accordance with the net effect. The new location, in contrast with the former position of the cell alone, should allow a classification of such new entity or group of new entities. If the particles attached to the cells are magnetic, then of course, according to current practice, the new entities can be captured by the use of a magnet. If mixed rapidly, unexpected results including complete capture of a population without adversely affecting the properties of cell under study occur.

Only three distinct populations of cells can be readily identified and enumerated from a blood sample by utilizing their inherent and unique properties of DC volume and opacity parameters heretofore stated. Additional steps, such as improved lysing systems, must be taken to enable the detection and enumeration of more populations. Of course, these additional populations represent subpopulations of the three basic ones referred to as lymphocytes, monocytes and granulocytes. The steps performed in accordance with this invention will demonstrate how subpopulations of these basic three populations are obtained.

Employing such simple aperture sensing techniques in combination with two or more biological particles, one can produce a unique and new position of the dot cluster representing a given population. This selective movement of populations on the dot plot or scattergram is reproducible and can be used to classify a population separate from the basic three populations.

The original and inherent combination of DC volume and opacity sensing techniques can be modified through the attachment of microscopic particles to selected individual cells. The selectivity is given the particles by the nature or specificity of the biological molecules, antibodies among others, employed as the coating on their surfaces. A population of cells alone, having no particles on their surface, may occupy a dot plot position no different from other populations or subpopulations and, henceforth, not be distinguishable from one another. The addition of particles having a selective attraction to a specific population of cells which one seeks to identify, enumerate, and study is possible using this approach. The selective addition of a sufficient mass of selective particles to a distinct population of interest results in the shifting of that population's dot plot location as a result of the new and unique combination of mass, volume and opacity.

The separation of specific cell populations is accomplished without materially affecting the properties of remaining cell populations. For example, the removal of erythrocytes or red blood cells (RBC's) from whole blood in accordance with this invention permits the measurement of T4 and/or T8 lymphocytes not otherwise possible with heretofore available chemical RBC lysing reagents. Ratios of the number of T4 versus T8 cells have been used to indicate immune deficiencies consistent with severe viral infections including the AIDS virus among others. The presence of specific receptors on the surface of cells can be used to classify a population into subsets, whose enumeration permits the detection of the onset of disease. For example, in the predominant forms of leukemia there is a sharp rise in peripheral blood lymphocytes. If the subpopulation of lymphocytes which is rapidly proliferating bears the T11 receptor, the patient is at risk of immune abnormalities. Further, if the subpopulation of T11 positive lymphocytes is T4 receptor bearing, then the patient is classified as that common in Japan. Moreover, if the T4 receptor subpopulations expanding is 2H4 positive, then the patient will not only demonstrate a tendency of multiple infections but acute leukemia as well for the T11, T4, 2H4 positive cell is the inducer of suppression and functionally inhibits the patient's ability to make antibodies. Therein, the patient is subject to multiple infections and must be treated for both leukemia and immune deficiency. K. Takatsuki, et al., GANN monograph on Cancer Research 28:13-22, 1982; C. Morimoto, et al., Coulter Japan Symposium, 1984; C. Morimoto, et al., Immunology 134 (3):1508-1515, 1985; C. Morimoto, et al., New England Journal of Medicine 316(2):67-71, 1987. The invention also applies to analyses of formed body suspensions such as bacteria and viruses among others.

This invention provides a method which combines electronic particle sensing technology and the specificity of selective biological molecules to enable a major advancement in the field of automated analyzers for clinical laboratory use, and for industrial applications. The detection of multiple leukocyte populations, and their relationship to one another in human peripheral blood is important in medical research and the diagnosis of human diseases. Such data are useful as a screening tool for identifying and classifying diseases, such as leukemia. Abnormal situations identified by implementation of the invention herein provides diagnostically relevant information in areas of study not limited only to detection of leukocyte populations as will be apparent from the specification and drawings hereof.

One of the most valuable features of this invention is that it employs the single rugged Coulter sensing operation. It is stable and does not require the complexity and expense of optical systems. The circuitry required for the addition of the RF generator and detector is economical, compact and reliable. A single aperture is all that is required, but the addition of a second or even a third aperture can enable a greater sample throuhput rate economically.

### Summary of the Invention

According to a first aspect of the present invention, a method of obtaining a multi-part WBC (white blood cell) population differential from a whole blood sample having at least a red blood cell population and WBC populations therein comprises the steps of:
shifting the neutrophil population electronic-sensing characteristic contribution with respect to the other WBC populations, by mixing with the sample microspheres having a neutrophil-specific monoclonal antibody bonded thereto; and
electronically sensing and counting at least the WBC populations of monocytes, lymphocytes, neutrophils, eosinophils and basophils using a low and a high frequency signal, thereby obtaining at least a five-part WBC differential.

According to a second aspect of the present invention, a method of obtaining a multi-part WBC population differential from a whole blood sample having at least a red blood cell population and WBC populations therein comprises the steps of:
(i) electronically sensing and counting at least the WBC populations of granulocytes, monocytes and lymphocytes using a low and a high frequency signal;
(ii) removing or shifting the neutrophil population contribution from the WBC populations, by mixing with the sample microspheres as defined in claim 1;
(iii) electronically sensing and counting at least the remaining WBC populations of monocytes, lymphocytes, eosinophils and basophils; and
(iv) comparing the two counts, to obtain a count of the WBC population of neutrophils and thereby at least a five-part WBC differential.

According to a third aspect of the present invention, a method of analysing a WBC population in a whole blood sample comprising a red blood cell population and WBC populations, of which a selected WBC population has at least two subsets, comprises the steps of:
removing or shifting at least one WBC population subset contribution from the selected WBC population by mixing with the sample microspheres having, bonded thereto, a monoclonal antibody specific to the at least one subset; and
electronically sensing and analysing the thussubtracted subset and the WBC population using a low and a high frequency signal, to determine at least one characteristic of the selected WBC population.

According to a fourth aspect of the present invention, a method for determining a WBC population or population subset in a whole blood sample comprises the steps of:
shifting at least one WBC population or subset electronic-sensing characteristic contribution with respect to other WBC population or subset population, by mixing with the sample microspheres having, bonded thereto, a monoclonal antibody specific to the at least one WBC population or subset; and
electronically sensing and directly counting the shifted population using a low and a high frequency signal, to obtain the contribution to the sample of the shifted population or subset.

According to a fifth aspect of the present invention, a method of analysing a WBC population or population subset in a whole blood sample comprising WBC populations including at least one WBC population subset comprises the steps of:
electronically sensing and counting the WBC populations and subsets thereof using a low and a high frequency signal, to give a first count;
removing or shifting at least one of the WBC populations or a subset thereof by mixing with the sample microspheres as defined in the fourth aspect of the invention;
electronically sensing and counting the remaining and/or shifted WBC populations using a low and a high frequency signal, to give a second count; and
comparing the first and second counts to obtain or derive either or both of the remaining WBC populations and the removed or shifted WBC population or subset population.

### Brief Description of Drawings

The preferred embodiments of this invention will now be described by way of example, with reference to the drawings accompanying this specification in which:
Fig. 1 is a schematic block diagram of a first cell population analyzer embodying a method of the invention;
Fig. 2 is a schematic block diagram of a second analyzer embodying a method of the invention;
Fig. 3 is one specific analyzer embodying a method of the invention and corresponding to Figs. 1 and 2;
Fig. 4 is a schematic block diagram of another analyzer embodying a method of the invention;
Fig. 5A and 5B are a scattergram of one set of results utilizing a prototype analyzer system similar to that illustrated with respect to Figs. 2 and 3;
Fig. 6 is a schematic block diagram of a further analyzer embodying a method of the invention;
Fig. 7 is a schematic block diagram of a still further analyzer embodying a method of the invention;
Figs. 8A and 8B, 9A and 9B, 10A and 10B and 11A and 11B are a scattergram of one set of results utilizing a prototype analyzer similar to that illustrated with respect to Figs. 6 and 7;
Fig. 12 is a schematic block diagram of a yet still further analyzer embodying a method of the invention; and
Fig. 13 is a scattergram of one set of results utilizing a prototype analyzer similar to that illustrated with respect to Fig. 12.

### Modes for Carrying Out the Invention

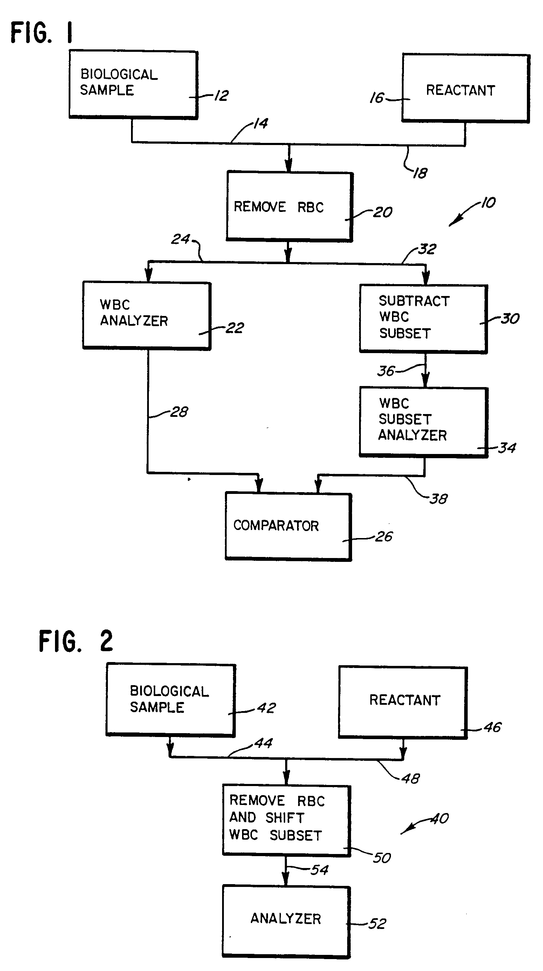

Referring to Fig. 1, a first embodiment of a cell population analyzing method of the present invention, and apparatus embodying that method, is designated generally by the reference numeral 10. The analyzer 10 includes a biological sample 12 which contains at least a first set of viable biological cells (not illustrated), such as in or from a whole blood sample. The cells of the biological sample 12 are to be involved in a biological reaction in a quantitative and/or qualitative determination or analysis. The sample 12 can include a buffer into which the cells are added.

The sample 12 is combined via a line 14 with at least one reactant 16 via a line 18. The red blood cells (RBC) then are removed form the mixture by a functionally designated RBC removing station 20. The RBC's can be removed from the mixture by the station 20 in a number of ways. The RBC's can be lysed by a lyse in the reactant 16. One such preferential lyse and a quench which can be utilized therewith is disclosed in U.S. Serial No. 025,303, entitled METHOD AND REAGENT SYSTEM FOR ISOLATION, IDENTIFICATION AND/OR ANALSIS OF LEUKOCYTES FROM WHOLE BLOOD SAMPLES, filed concurrently herewith, which is incorporated herein by reference. The reactant 16 can be or include a plurality of magnetic microspheres with an antibody specific to the RBC's bound to the microspheres (not illustrated). In this example, the particular red blood cell specific antibody utilized is disclosed in Application Serial No. 799,489, filed November 19, 1985, entitled MONOCLONAL ANTIBODY FOR RECOVERY OF LEUKOCYTES IN HUMAN PERIPHERAL BLOOD AND METHOD OF RECOVERY EMPLOYING SAID MONOCLONAL ANTIBODY, which is incorporated herein by reference. The reactant 16 also can include a buffer in addition to or in place of the sample buffer. The reactant 16 further can be a combination of the preferential RBC lyse and the RBC specific microspheres.

Once the RBC's substantially are removed from the mixture, a portion of the mixture is fed into a white blood cell (WBC) analyzer 22 via a line 24. The WBC analyzer 22 at least counts the number of WBC's in the mixture. The WBC analyzer 22 also can measure one or more volume or opacity parameters of the WBC's. The results from the analyzer 22 are fed to a comparator 26 via a line 28.

A second portion of the RBC deleted mixture is fed to a WBC subset subtracting station 30 via line 32. The WBC's can be subtracted from the mixture in a number of ways. Microspheres with a monoclonal antibody specific to one of the WBC subsets bound thereto can be added to the mixture. Non-magnetic microspheres can be bound to the WBC's to change or shift the resultant opacity or volume parameters of the cells. Magnetic microspheres also can be bound to the WBC's which then can be removed from the mixture by a magnetic field.

The mixture with the WBC subset population removed or with one or more parameters changed then is fed to a WBC subset analyzer 34 via a line 36. The analyzer 34 can be identical to the analyzer 22. The results of the analyzer 34 then are fed to the comparator 26 via a line 38. The comparator 26 then can compare the WBC results from the analyzer 22 with the modified results from the analyzer 34 to determine at least one characteristic of the selected white blood cell population, such as the number of cells in a particular range.

Referring to Fig. 2, a second embodiment of a cell population analyzing method of the present invention, and apparatus embodying that method, is designated generally by the reference numeral 40. The analyzer 40 includes a biological sample 42 which again contains at least a first set of viable biological cells (not illustrated), such as in or from a whole blood sample. The cells of the biological sample 42 are to be involved in a biological reaction in a quantitative and/or qualitative determination or analysis. The sample 42 again can include a buffer into which the cells are added.

The sample 42 is combined via a line 44 with at least one reactant 46 via a line 48. In the analyzer 40, the RBC's are removed from the mixture and simultaneously at least one characteristic of at least one WBC subset is changed or shifted by a functionally designated RBC removing and WBC shifting station 50. As stated above, the RBC's can be removed from the mixture by the station in a number of ways, previously enumerated with respect to the station 20. Simultaneously, in the same mixture portion, the WBC's are bound to, generally non-magnetic, microsphere to change or shift the resultant opacity and/or volume parameters of the cells.

The mixture with the RBC's removed and the WBC subset population shifted then is fed to an analyzer 52 via a line 54. The analyzer 52 can be substantially identical to the analyzer 22. The analyzer 40 thus provides a fast, direct analysis of at least one characteristic of a selected WBC population or whole blood subset.

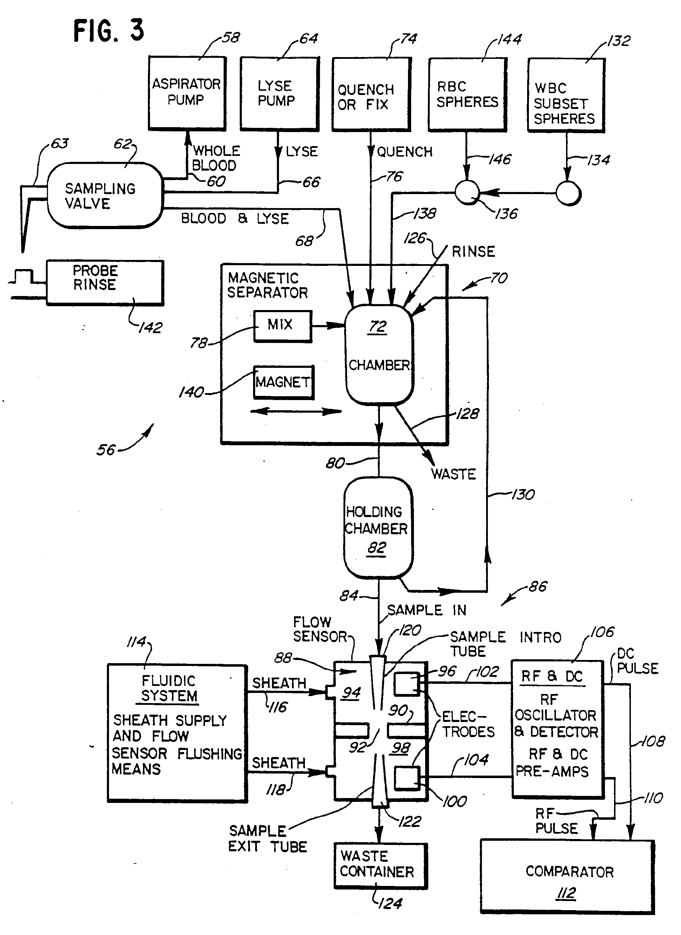

One specific embodiment of an analyzer instrument embodying a method of the invention and which can accomplish the analyzing methods of the first and second analyzer 10 and 40, is designated generally by the reference numeral 56 in Fig. 3.

In the instrument 56, only one specific enumeration is illustrated, which can be varied in almost endless detail in accordance with the principles of the invention. Further, the instrument 56 is shown in generally functional detail and the specific embodiments can be structurally implemented in many known ways.

The instrument 56 includes an aspirator pumping mechanism 58 which is utilized to draw the biological sample of interest, for example the sample 12 or 42 into the instrument 56. The aspirator 58 is coupled via a line 60 to a sampling valve 62, which can be coupled to a sample probe 63. A lyse pump 64 can include the lyse, such as part of the reactant 18 or 46 and is also coupled to the valve 62 via a line 66. The valve 62 and the pump 58 can aspirate the biological sample 12 or 42 along with the lyse via the pump 64 when appropriate.

The reactant mixture or the biological sample itself, then is fed via a discharge line 68 into a mixing apparatus 70. The mixer 70 includes a mixing chamber 72 into which the sample or reactant is fed. At this point the operation of the analyzer 10 and 40 differ and hence will be described separately.

In the case of the analyzer 10, if the RBC's have been lysed by the lyse from the pump 64, then when the reaction is completed a quench or fix is supplied from a station 74 via a line 76. The reaction is completed. The reaction can be assisted by mixing the lyse and the sample in the chamber 72 as illustrated functionally at 78.

Specific details of an appropriate mixing apparatus 70, which can be utilized herein are disclosed in U.S. Serial No. 025,337, entitled METHOD AND APPARATUS FOR RAPID MIXING OF SMALL VOLUMES FOR ENHANCING BIOLOGICAL REACTIONS, filed concurrently herewith, which is incorporated herein by reference. By utilizing the mixer 70 the reactions are greatly enhanced in speed without significantly damaging the properties of interest of the cells, such as, can occur by raising the reaction temperature. Further, the reactions generally are completed in significantly less than a minute, generally on the order of fifteen seconds or less. This allows a rapid analysis of the automatic high volume analyzer instrument 56.

The quenched reactant with the RBC's removed by the lyse (as from the station 20) then is fed via a line 80 to a holding chamber 82, which in this case will hold a second portion of the mixture. A first portion of the mixture will be fed from the chamber 82 via a line 84 to a WBC analyzer 86 (i.e. analyzer 22). The analyzer 86 can be of many physical types in accordance with the counting and sizing techniques described by Wallace H. Coulter in U.S. Patent No. 2,656,508 and embodied in the numerous commercial blood cell counter of the assignee, Coulter Electronics, Inc.

The analyzer 86, in general, includes a flow sensor or sensing chamber 88. The chamber 88 includes a transducer 90 which has an aperture 92 therethrough. The chamber 88 includes a first portion 99 which has a first electrode 96 in contact with the fluid therein.

The chamber portion 94 and the electrode 96 communicate through the aperture 92 with a second chamber portion 98 having a second electrode 100 therein.

The electrodes 96 and 100 are coupled via reactive leads 102 and 104 to an RF/DC source and sensing circuit 106. The circuit 106 couples both a DC, or low frequency current or signal and a high frequency signal between the electrodes 96 and 100.

The low frequency signal is utilized to sense the amplitude of a signal pulse caused by a cell passing through the aperture 92. The high frequency signal is utilized to obtain the electrical opacity of the same cell passing through the aperture 92.

The measuring of the electrical opacity of cells was described by Wallace H. Coulter and Walter R. Hogg in U.S. Patent No. 3,502,974 and several patents and publications of the assignee, Coulter Electronics, Inc., since that patent. One specific circuit which can be utilized herein is disclosed in U.S. Serial No. 921,654, entitled PARTICLE ANALYZER FOR MEASURING THE RESISTANCE AND REACTANCE OF A PARTICLE, filed October 21, 1986, which is incorporated herein by reference.

The signals generated by the circuit 106 from the sensed cells are coupled via a DC signal lead 108 and an RF signal lead 110 to a comparator 112 (like the comparator 26). The comparator 112 can hold the signal generated from the first portion, i.e. those without the WBC subset substracted, for a comparison with the results from the second portion to be described.

The analyzer 86 can include a sheath flow to focus the cells in the sensor 88, in the well known manner. The sheath flow can be provided by a fluidic system 114, coupled to the sensor 88 by a pair of lines 116 and 118 in a known manner. The sample reaction mixture can be fed into the sensor 88 via an introduction tube 120 and can be fed from the sensor 88 via an exit tube 122 into a waste container 124.

While the first portion of the mixture was being analyzed in the analyzer 86, the second portion is held in the chamber 82, while the mixer 72 is cleaned or flushed via a rinse line 126 and exhausted through a waste line 128. Once the chamber 72 is cleaned, the second portion is fed back into the chamber 72 via a line 130. Like the station 30, the WBC subset now is subtracted by adding the WBC microspheres from a station 132 via a line 134, a valve 136 and a chamber line 138.

The WBC microspheres are mixed with the second portion by the mixing mechanism 78. If the WBC microspheres are non-magnetic, the reaction mixture with the bound WBC microspheres is fed via the line 80, the chamber 82 and the line 84 into the analyzer 86, (i.e. the analyzer 34), wherein the second portion is analyzed like the first portion and the results then are compared in the comparator 112 (i.e. the comparator 26). At least one of the WBC subset cell parameters is changed in the second portion, such as the cell opacity by the WBC subset bound microspheres to provide the changed results which then can be analyzed.

If the WBC microspheres are magnetic, then the WBC subset bound thereto are removed by a magnetic field during and/or after the mixing process by a magnetic field or magnet 140. The field can be provided by electromagnetic means or by the magnet 140 being physically moved with respect to the chamber 72 to capture the magnetically bound WBC subset. The second portion without the bound WBC subset then is fed via the line 80, the chamber 82 and line 84 to the analyzer 86 in the manner previously described to obtain the analysis (like the analyzer 34).

The instrument 56 then is prepared to take the next sample for the next analysis. The probe 63 can be cleaned by a probe rinse mechanism 142 and the lines and chambers 72 and 82 can be flushed in a conventional manner. Each analysis of the succeeding sample mixture is obtained in a rapid and automatic fashion. The period between the analysis of succeeding sample mixtures can be on the order of minutes or less.

In operating the analyzer instrument 56, like the analyzer 40, the reaction mixture with the RBC lyse/reactant 46 and the sample 42 is mixed in the chamber 72 along with non-magnetic WBC microspheres from the station 132, which bind to one of the WBC subsets. The quench 74 is added to the reactive mixture which then is fed via the line 80, the chamber 82 and the line 84 to the WBC analyzer 86 for analysis (i.e. like the analyzer 52).

Alternatively to the utilization of the lyse, in either of the analyzers 10 and 40, the sample 12 or 42 can be fed to the mixer 70 via the valve 62 without any lyse. in this case the RBC's can be removed magnetically by utilizing the microspheres with the RBC specific antibody bound thereto from an RBC microsphere station 144 and fed to the valve 136 via a line 146 and hence to the chamber 70 via the line 138. Where no lyse is utilized, the bound RBC's are magnetically removed by the magnet 140 after mixing in a manner substantially identical to the magnetically bound WBC's described above.

Further, in a second case to promote the speed of the reaction, a reaction mixture of the sample with both the RBC lyse and with the RBC magnetic beads can be utilized. The reaction mixture is mixed, the lyse is quenched and the bound RBC's are magnetically removed and then the WBC's are analyzed as previously described.

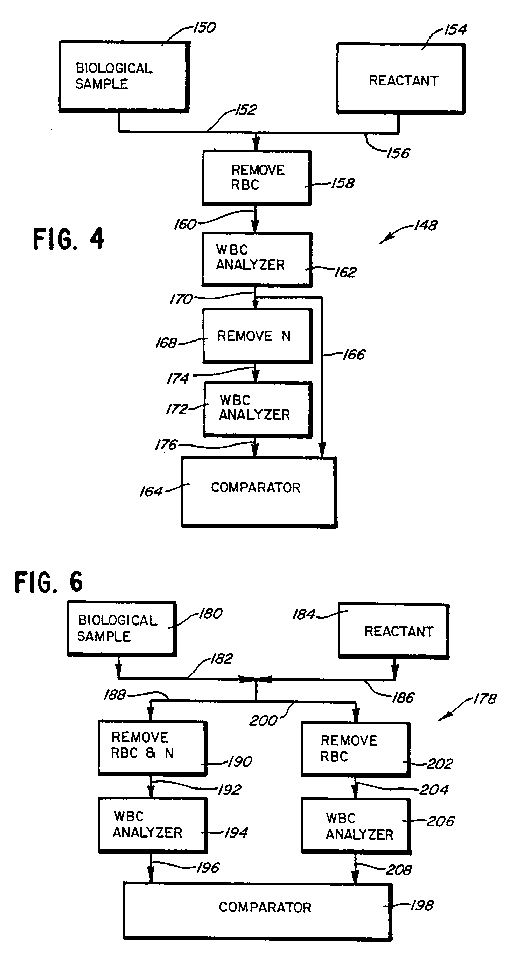

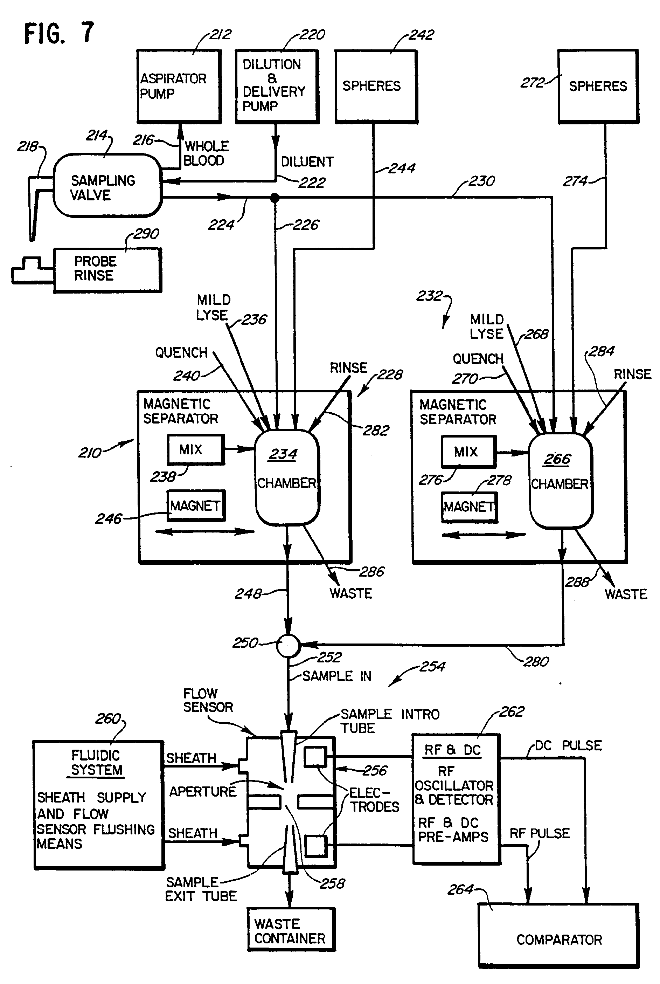

Referring now to Fig. 4, another embodiment of a cell population analyzing method of the present invention, and apparatus embodying that method, is designated generally by the reference numeral 148. The analyzer 148 includes a biological sample 150 which again contains at least a first set of viable biological cells, such as in or from a whole blood sample. The sample 150 again can include a buffer into which the cells are added.

The sample 150 is combined via a line 152 with at least one reactant 154 via a line 156. The RBC's then are removed as above described by a functionally designated RBC removing station 158. The reaction mixture with the RBC's removed is fed via a line 160 into a WBC analyzer 162. The results from the analyzer 162 are fed to a comparator 164 via a line 166, providing a three-part WBC differential with results for monocytes (M), lymphocytes (L) and granulocytes (G).

The mixture then is fed to a neutrophil (N) functionally designated removal station 168 via a line 170. The N's can be removed from the mixture by shifting or changing one parameter, such as opacity, or by magnetic removal, both as described above. In this example, the particular N specific antibody utilized is disclosed in U.S. Serial No. 938,864, entitled MONOCLONAL ANTIBODY SPECIFIC TO NEUTROPHILS, filed December 8, 1986.

The mixture with the N's removed or shifted then is fed to another WBC analyzer 172 via a line 174. The results of the analyzer 172 are fed to the comparator 164 via a line 176. The results of the analyzer 172 are utilized to obtain a four-part WBC differential with results again for M's and L's, but now in addition since the N's are shifted or removed results for eosinophils (E) and basophils (B) are obtained. The two analytical results from the analyzers 162 and 172 then can be compared by the comparator 164 to form a five-part WBC differential. Specifically, subtracting the number of B's and E's from the number of Gr's results in the number of the removed N's.

Referring now to Figs. 5A and 5B, two sets of scattergram results are illustrated obtained from a whole blood sample utilizing a prototype analyzing method similar to the analyzer 148. The biological sample 150 was a 20 microliter sample of whole blood, which was combined with 40 microliters of the magnetic microspheres with the RBC specific antibody bound thereto combined with 140 microliters of buffer solution to form the reactant 154. The reaction mixture was mixed for 15 seconds and placed in a magnetic field for 10 seconds in the station 158. The mixture with the RBC's removed was analyzed by the analyzer 162 as illustrated in the scattergram of Fig. 5A resulting in counts of L's of 45.6 (1), M's of 5.6 (2) and Gr's of 48.7 (3).

The mixture then is combined in the station 168 with 10 microliters of magnetic microspheres with the N specific antibody bound thereto. The mixture is mixed 30 seconds and then placed in a magnetic field for 10 seconds. The mixture with the N's then removed was fed to the analyzer 176 which resulted in the scattergram of Fig. 5B resulting in counts of L's of 81.0 (1), M's of 0.6 (2), E's of 11.0 (3) and B's of 1.8 (4). The comparator 164 then provides the five-part WBC differential of counts of 45.6 L's, 5.6 M's, 41.6 N's, 6.0 E's and 1.2 B's. This corresponds to a standard microscopic five-part WBC differential utilizing Wright stain on the sample on a slide resulting in counts of 44.0 L's, 3.4 M's, 45.0 N's, 6.1 E's and 0.4 B's.

Fig. 6 illustrates a further embodiment of a cell population analyzing method of the present invention, and apparatus embodying that method, designated generally by the reference nuemral 178. The analyzer 178 includes a biological sample 180 which again contains at least a first set of viable biological cells and also can include a buffer.

The sample 180 is combined via a line 182 with a reactant 184 via a line 186. Functionally illustrated, a first portion of the mixture is fed via a line 188 to a functionally designated RBC and N removing station 190. The RBC's and N's are removed or shifted as described before and the first portion is fed via a line 192 to a WBC analyzer 194.

This provides a result from the analyzer 194 which is fed via a line 196 to a comparator 198. The result includes the above-referenced four-part differential including M's, L's, E's and B's.

At the same time, a second portion of the mixture of the sample 180 and the reactant 184 is fed via a line 200 to a functionally designated RBC removal station 202. The mixture with the RBC's removed is fed via a line 204 to another WBC analyzer 206. The results of the analyzer 206 are fed to the comparator 198 via a line 208. The results of the analyzer 206 directly include the above-referenced three-part WBC differential including M's, L's and Gr's. The results of the analyzers 194 and 206 then are compared by the comparator 198 to provide the five-part WBC differential.

A still further analyzing instrument incorporating the method and apparatus of the analyzer 178 is designated generally by the reference numeral 210 in Fig. 7. Again, only one specific hardware enumeration has been illustrated, but like tha analyzing instrument 56, the analyzing instrument 210 can be implemented in numerous configurations.

The instrument 210 includes an aspirator purging mechanism 212 which is coupled to a sampling valve 214 via a line 216. The valve 214 can include a sample probe 218 to aspirate the biological sample of interest, such as the sample 180. A diluent delivery pump 220 is coupled to the valve 214 via a line 222 to provide a diluent for the sample, such as a whole blood sample, when desired. A first portion of the mixture then is coupled via a line 224 and a line 226 to a first mixing apparatus 228. At the same time, a second poation of the mixture is fed via the line 224 and a line 230 to a second mixing apparatus 232.

The mixer 228 (comparable to the station 190) is substantially identical to the mixer 232 (comparable to the station 202) and will be described first. The mixer 228 includes a mixing chamber 234 into which the first mixture portion is fed. The mixer 228 includes all of the various options above described and can include a lyse input line 236 for the RBC lyse if desired.

If the lyse is utilized, after mixing as illustrated functionally at 238, then the quench is added via a quench line 240. At the same time, the N's are being removed by the addition of the appropriate magnetic or non-magnetic microspheres with the N specific antibody bound thereto from a source of microspheres 242 fed to the chamber 234 via a line 244. If magnetic microspheres are utilized for the N's or the RBC's, then a magnet 246 or magnetic field is utilized to remove the magnetically bound cells.

The mixed and quenched (where necessary) mixture then is fed via a line 248 through a valve 250 and line 252 to a WBC analyzer 254 (i.e. analyzer 194). The analyzer 254 is the same as the analyzer 86 and will not be described again in such detail. Again, the analyzer 254 includes a sensing chamber 256 with an aperture 258 therein through which the mixture and cells pass. A sheath flow fluidic system 260 can be coupled to the chamber 256. The signals generated by the cells are detected by an RF/DC source and sensing circuit 262 whose outputs are fed to a comparator 264, as previously described.

Concurrently, the second mixture portion is fed into a mixing chamber 266. In the second portion, only the RBC's are removed (i.e. like the station 202) and the RBC's can be removed by the RBC lyse fed into the chamber 266 via a line 268. The lyse is mixed with the sample and then a quench is added via a quench line 270. Alternatively the RBC's can be removed by magnetic microspheres having the RBC specific antibody bound thereto from a microsphere source 272 fed into the chamber 266 via a line 274. The microspheres are fixed, functionally at 276, and then the magnetically bound RBC microspheres are removed by a magnet 278.

The RBC removed mixture then is fed via a line 280 to the valve 250 and via the line 252 to the analyzer 254 to obtain the above-mentioned results. The mixers 228 and 232 include appropriate respective rinse lines 282 and 284 and waste lines 286 and 288 and a probe rinse 290 to cleanse the instrument 210 prior to aspirating the next sample or samples for analyzing.

Figs. 8A and 8B illustrate scattergram results obtained from a whole blood sample utilizing an analyzing method similar to the analyzer 178. In this example, 20 microliters of whole blood form the sample 180, while 40 microliters of magnetic microspheres with the RBC specific antibody bound thereto combined with 140 microliters of buffer solution form the reactant 184. A portion of the mixture is mixed for 20 seconds in the station 202 and then placed in a magnetic field for 10 seconds. The RBC removed mixture then is analyzed in the analyzer 206 resulting in the scattergram of Fig. 8A which provides a count of L's 29.4 (1), M's 8.1 (2) and Gr's 62.4 (3).

At the same time, another portion of the same mixture is combined with 10 microliters of magnetic microspheres with the N specific antibody bound thereto to remove the RBC's and N's in the station 190. The mixture is mixed for 30 seconds, then placed in a magnetic field for 10 seconds. The mixture with the N's and RBC's removed then is analyzed by the analyzer 194 resulting in the scattergram of Fig. 8B which provides a count of L's 73.5 (1), M's 21.7 (2), E's 3.4 (3) and B's 1.4 (4). The two counts are compared in the comparator 198, resulting in a five-part WBC differential count of L's 29.4, M's 8.0, N's 60.8, E's 1.2 and B's 0.6. A microscope comparison again was made resulting in counts of L's 29.4, M's 5.0, N's 65.0, E's 1.0 and B's of less than 1.0.

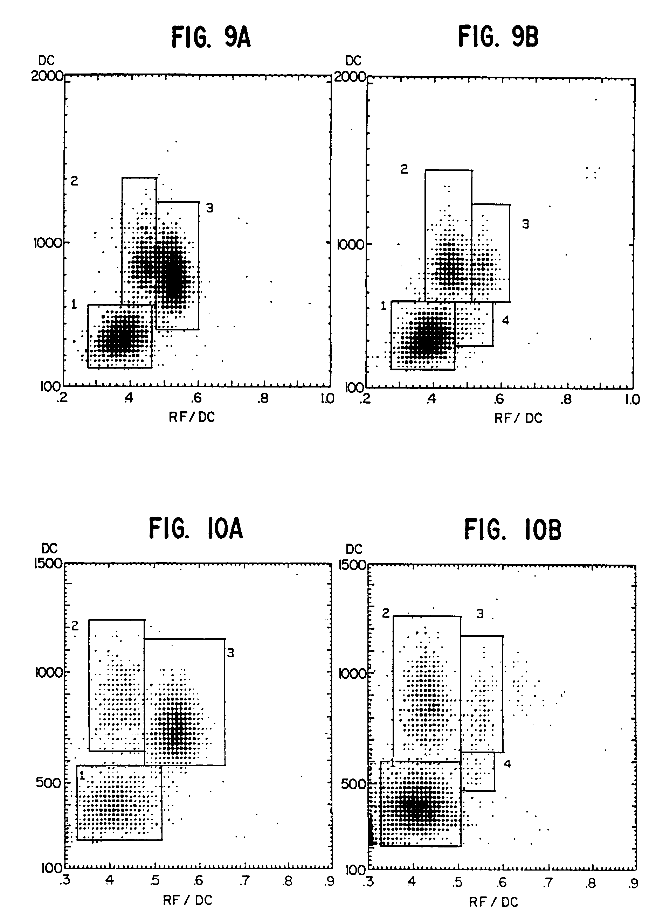

Figs. 9A and 9B show scattergram results of a fivepart WBC differential example similar to that of Figs. 8A and 8B. A 20 microliter sample of whole blood was analyzed in the same steps described with respect to Figs. 8A and 8B resulting in the scattergram of Fig. 9A providing a count of L's 35.4 (1), M's 14.6 (2) and Gr's 50.0 (3). The scattergram of Fig. 9B provides a count of L's 66.4 (1), M's 25.0 (2), E's 6.6 (3) and B's 2.0 (4). The resulting five-part WBC differential results in counts of 35.4 L's, 14.6 M's, 45.5 N's, 3.5 E's and 1.1 B's was compared to a microscope count of 36 L's, 11 M's, 49 N's, 3 E's and 1 B.

Figs. 10A and 10B show scattergram results of a five-part WBC differential again similar to that of Figs. 8A, 8B and 9A, 9B, however, in this example, lyse was utilized. In this example, 20 microliters of whole blood was combined with 80 microliters of buffer and 240 microliters of the RBC preferential lyse above referenced. The mixture is mixed for 6 seconds and then a quench is added. The time period is significant, because the lyse left unquenched for a period of time greater than about 10 seconds will start to affect the significant properties of the WBC's. The mixture with the RBC's removed is analyzed to provide the scattergram of Fig. 10A resulting in counts of L's 25.7 (1), M's 9.6 (2) and Gr's 65.0 (3).

A second portion of the mixture including a second 20 microliter sample of the whole blood is combined with 120 microliters of buffer and 10 microliters of magnetic microspheres with the N specific antibody bound thereto and mixed for 30 seconds and then placed in a magnetic field for 10 seconds. The RBC preferential lyse then is added to the N removed mixture which then is mixed for 6 seconds before it is quenched. The resulting scattergram Fig. 10B results in percentage counts of L's 74.6 (1), M's 21.6 (2), E's 2.9 (3) and B's 0.8 (4). The resulting five-part WBC differential results in percentage counts of L's 25.6, M's 9.6, N's 63.5, E's 1.06 and B's 0.3. Again a microscope comparison resulted in counts of L's 29.4, M's 5.0, N's 65.0, E's 1.0 and B's of less than 1.

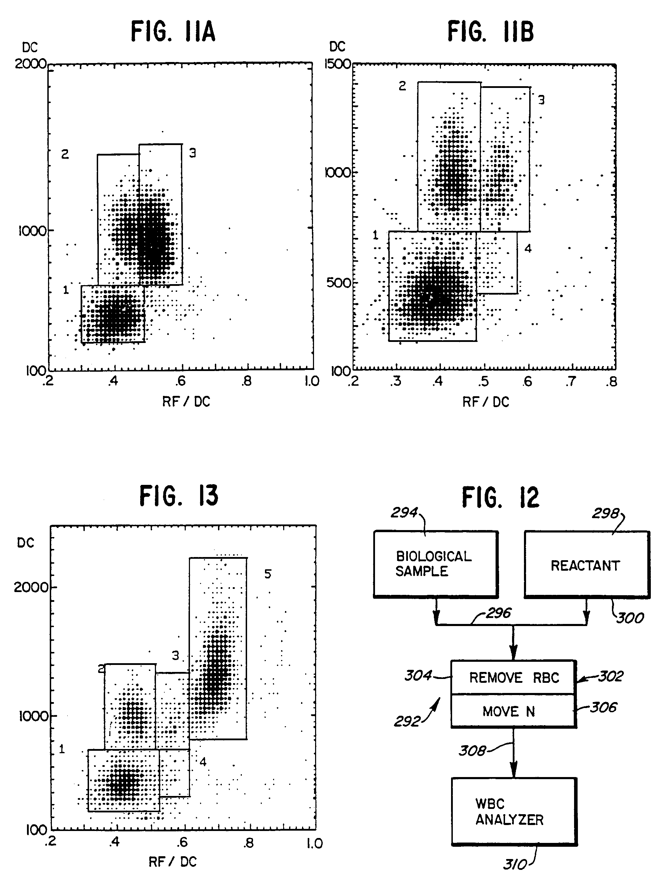

Another example of scattergram results of a five-part WBC differential similar to that of Figs. 10A and 10B is illustrated in Figs. 11A and 11B. A sample of whole blood had two samples simultaneously analyzed in the same steps described with a respect to Figs. 10A and 10B. The scattergram of Fig. 11A provides a count of L's 31.9 (1), M's 17.6 (2) and Gr's 50.4 (3). The scattergram of Fig. 11B provides a count of L's 67.1 (1), M's 24.1 (2), E's 7.6 (3) and B's 1.2 (4). The resulting five-part WBC differential results in counts of 31.9 L's, 11.4 M's, 46.0 N's, 3.6 E's and 0.7 B's as compared to a microscope count of 36 L's, 11 M's, 49 N's, 3 E's and 1 B.

A yet still further embodiment of a cell population analyzing method of the present invention, and apparatus embodying that method, is designated generally by the reference numeral 292 in Fig. 12. The analyzer 292 includes a biological sample 294, again including at least a first set of viable biological cells and including a buffer if desired.

The sample 294 is combined via a line 296 with at least one reactant 298 via a line 300. In the analyzer 292, the RBC's are removed and the N's are shifted sequentially or simultaneously in a functionally designated station 302. The RBC remove function is designated 304 and the N move or shift portion is designated 306 to indicate that the functions can be performed simultaneously or sequentially. The RBC's can be removed magnetically or with lyse or with a combination of the two as previously described. The N's are removed or shifted by adding microspheres having an N specific antibody bound thereto to the mixture.

Once the RBC's are removed and the N's are moved or shifted, then the resulting mixture is fed via a line 308 to an analyzer 310. In this case, the N's are shifted sufficiently from the patterns of the E's and B's that a five-part WBC differential of M's, L's, E's, B's and N's is directly obtained. The functions of the analyzer 292 can be performed on either of the instruments 56 and 210 or minor variations thereof.

The scattergram results of one example of a direct five-part WBC differential in accordance with the analyzer 292 is illustrated in Fig. 13. In this example, the biological sample 294 is 20 microliters of a whole blood sample and the reactant 298 is 10 microliters of non-magnetic microspheres with the N specific antibody bound thereto combined with 100 microliters of buffer and mixed in the substation 306 for 30 seconds. The RBC preferential lyse, 240 microliters thereof, then is added to the mixture which is mixed in the substation 304 for 6 seconds after which the quench is added. The RBC removed and N shifted mixture then is analyzed by the analyzer 310 resulting in the scattergram of Fig. 13 which provides a direct count of 29.6 L's, 13.6 M's, 52.2 N's, 3.4 E's and 1.06 B's as compared to a microscope determination of 35 L's, 5 M's, 56 N's, 4 E's and no B's. In this particular example, the whole blood sample was also analyzed on a general cell counting instrument of Coulter Electronics, Inc., which resulted in 29 L's, 11.1 M's and 59.9 Gr's (N's, E's and B's).

Many modifications and variations of the present invention are possible in light of the above teachings. The samples 12, 42, 150, 180 and 294 can include whole blood, human body fluids containing cells, or other fluids containing formed bodies, such as bacteria, viruses and fungi. The volumes of microspheres specified are stated in weight of microspheres per volume of diluent. It is therefore, to be understood that within the scope of the appended claims, the invention may be practiced otherwise than as specifically described.

## Claims

1. A method of obtaining a multi-part WBC (white blood cell) population differential from a whole blood sample having at least a red blood cell population and WBC populations therein, which comprises the steps of:
shifting the neutrophil population electronic-sensing characteristic contribution with respect to the other WBC populations, by mixing with the sample microspheres having a neutrophil-specific monoclonal antibody bonded thereto; and
electronically sensing and counting at least the WBC populations of monocytes, lymphocytes, neutrophils, eosinophils and basophils using a low and a high frequency signal thereby obtaining at least a five-part WBC differential.

2. A method of obtaining a multi-part WBC (white blood cell) population differential from a whole blood sample having at least a red blood cell population and WBC populations therein, which comprises the steps of:
(i) electronically sensing and counting at least the WBC populations of granulocytes, monocytes and lymphocytes using a low and a high frequency signal;
(ii) removing or shifting the neutrophil population contribution from the WBC populations, by mixing with the sample microspheres as defined in claim 1;
(iii) electronically sensing and counting at least the remaining WBC populations of monocytes, lymphocytes, eosinophils and basophils; and
(iv) comparing the two counts, to obtain a count of the WBC population of neutrophils and thereby at least a five-part WBC differential.

3. A method according to claim 2, wherein step (i) is conducted on a first portion of the sample, and steps (ii) and (iii) are conducted on a second portion of the sample.

4. A method of analysing a WBC (white blood cell) population in a whole blood sample comprising a red blood cell population and WBC populations, of which a selected WBC population has at least two subsets, which comprises the steps of:
removing or shifting at least one WBC population subset contribution from the selected WBC population by mixing with the sample microspheres having, bonded thereto, a monoclonal antibody specific to the at least one subset; and
electronically sensing and analysing, the thus- substracted subset and the WBC population using a low and a high frequency signal, to determine at least one characteristic of the selected WBC population.

5. A method for determining a WBC (white blood cell) population or population subset in a whole blood sample, which comprises the steps of:
shifting at least one WBC population or subset electronic-sensing characteristic contribution with respect to other WBC population or subset population, by mixing with the sample microspheres having, bonded thereto, a monoclonal antibody specific to the at least one WBC population or subset; and
electronically sensing and directly counting the shifted population using a low and a high frequency signal, to obtain the contribution to the sample of the shifted population or subset.

6. A method of analysing a WBC (white blood cell) population or population subset in a whole blood sample comprising WBC populations including at least one WBC population subset, which comprises the steps of:
electronically sensing and counting the WBC populations and subsets thereof using a low and a high frequency signal, to give a first count;
removing or shifting at least one of the WBC populations or a subset thereof by mixing with the sample microspheres as defined in claim 5;
electronically sensing and counting the remaining and/or shifted WBC populations using a low and a high frequency signal, to give a second count; and
comparing the first and second counts to obtain or derive either or both of the remaining WBC populations and the removed or shifted WBC population or subset population.

7. A method according to any preceding claim, which comprises the additional prior step of removing the red blood cell population from the sample, or portions thereof, without adversely affecting the relevant qualities and/or quantities of the WBC populations.

8. A method according to any preceding claim, wherein the sensing and counting or analysing is of the volume and opacity of cells.

## Patentansprüche

1. Verfahren zum Erhalten eines vielteiligen WBC-(weiße Blutzelle)-Populationsdifferential von einer Vollblutprobe mit mindestens einer roten Blutzellenpopulation und WBC-Populationen darin, welches Verfahren die Schritte umfaßt:
Verschieben des charakteristischen Beitrags der Neutrophilenpopulation zum elektronischen Abtasten hinsichtlich der anderen WBC-Populationen, indem die Probe mit Mikrokugeln vermischt wird, auf welchen ein neutrophilspezifischer, monoklonaler Antikörper gebunden ist; und
elektronisches Abtasten und Zählen mindestens der WBC-Populationen von Monozyten, Lymphozyten, Neutrophilen, Eosinophilen und Basophilen, wobei ein niedrig- und ein hochfrequentes Signal verwendet werden, wodurch mindestens ein fünffteiliges WBC-Differential erhalten wird.

2. Verfahren zum Erhalten eines vielteiligen WBC-(weiße Blutzelle)-Populationsdifferentials von einer Vollblutprobe mit mindestens einer roten Blutzellenpopulation und WBC-Populationen darin, welches Verfahren die Schritte umfaßt:
(i) elektronisches Abtasten und Zählen von mindestens der WBC-Populationen von Granulozyten, Monozyten und Lymphozyten, wobei ein niedrig- und ein hochfrequentes Signal verwendet werden;
(ii) Entfernen oder Verschieben des Neutrophilpopulationsbeitrags von den WBC-Populationen, indem die Probe mit Mikrokugeln, wie in Anspruch 1 definiert, vermischt wird;
(iii) elektronisches Abtasten und Zählen von mindestens der verbleibenden WBC-Populationen von Monozyten, Lymphozyten, Eosinophilen und Basophilen; und
(iv) Vergleichen der zwei Zählungen, um eine Zählung der WBC-Population von Neutrophilen und dadurch mindestens ein fünfteiliges WBC-Differential zu erhalten.

3. Verfahren nach Anspruch 2, wobei der Schritt (i) mit einem ersten Teil der Probe ausgeführt wird, und wobei die Schritte (ii) und (iii) mit einem zweiten Teil der Probe ausgeführt werden.

4. Verfahren zum Analysieren einer WBC-(weiße Blutzelle)-Population in einer Vollblutprobe, welche eine rote Blutzellenpopulation und WBC-Populationen umfaßt, von welchen eine gewählte WBC-Population mindestens zwei Untergruppen besitzt, welches Verfahren die Schritte umfaßt:
Entfernen oder Verschieben von mindestens eines WBC-Populationsuntergruppenbeitrags von der gewählten WBC-Population, indem die Probe mit Mikrokugeln vermischt wird, auf welche ein monoklonaler Antikörper gebunden ist, der für mindestens eine Untergruppe spezifisch ist; und
elektronisches Abtasten und Analysieren der auf diese Weise subtrahierten Untergruppe und WBC-Population unter Venvendung eines niedrig- und eines hochfrequenten Signals, um mindestens eine charakteristische Eigenschaft der gewählten WBC-Population zu bestimmen.

5. Verfahren zum Bestimmen einer WBC-(weiße Blutzelle)-Population oder einer Populationsuntergruppe in einer Vollblutprobe, welches Verfahren die Schritte umfaßt:
Verschieben von mindestens eines charakteristischen Beitrags einer WBC-Population oder Untergruppe zum elektronischen Abtasten hinsichtlich einer anderen WBC-Population oder Untergruppenpopulation, indem die Probe mit Mikrokugeln vermischt wird, auf welche ein monoklonaler Antikörper gebunden ist, der für mindestens eine WBC-Population oder Untergruppe spezifisch ist; und
elektronisches Abtasten und direktes Zählen der verschobenen Population, wobei ein nieder- und ein hochfrequentes Signal venvendet werden, um den Beitrag zur Probe der verschobenen Population oder Untergruppe zu erhalten.

6. Verfahren zum Analysieren einer WBC-(weiße Blutzelle)-Population oder Populationsuntergruppe in einer Vollblutprobe, umfassend WBC-Populationen mit mindestens einer WBC-Populationsuntergruppe, welches Verfahren die Schritte umfaßt:
elektronisches Abtasten und Zählen der WBC-Populationen und ihrer Untergruppen, wobei ein nieder- und ein hochfrequentes Signal venvendet werden, um ein erste Zählung zu erhalten;
Entfernen oder Verschieben von mindestens einer der WBC-Populationen oder einer Untergruppe davon, indem die Probe mit Mikrokugeln, wie in Anspruch 5 definiert, vermischt wird;
elektronisches Abtasten und Zählen der verbleibenden und/oder verschobenen WBC-Populationen unter Anwendung eines nieder- und eines hochfrequenten Signals, um eine zweite Zählung zu erhalten; und
Vergleichen der ersten und der zweiten Zählung, um eine oder beide der verbleibenden WBC-Populationen und der entfernten oder verschobenen WBC-Population oder Untergruppenpopulation zu erhalten oder abzuleiten.

7. Verfahren nach einem vorhergehenden Anspruch, welches den zusätzlichen früheren Schritt einer Entfernung der roten Blutzellenpopulation von der Probe oder ihrer Teile umfaßt, ohne die relevanten Qualitäten und/oder Quantitäten der WBC-Populationen nachteilig zu beeinflussen.

8. Verfahren gemäß einem vorhergehenden Anspruch, wobei nach dem Volumen und der Zellenopazität abgetastet und gezählt oder analysiert wird.

## Revendications

1. Méthode d'obtention d'un différentiel de population de WBC (globules blancs) multipartie à partir d'un échantillon de sang entier ayant au moins une population de globules rouges et des populations de WBC à l'intérieur qui comprend les étapes suivantes :
déplacer la contribution aux caractéristiques de détection électronique de la population de neutrophiles par rapport aux autres populations de WBC, en mélangeant avec les microsphères échantillons ayant un anticorps monoclonal spécifique des neutrophiles fixé à celles-ci; et
détecter et compter électroniquement au moins les populations de WBC de monocytes, lymphocytes, neutrophiles, éosinophiles et basophiles en utilisant un signal à haute et basse fréquences pour obtenir de ce fait au moins un différentiel de WBC à cinq parties.

2. Méthode d'obtention d'un différentiel de population de WBC (globules blancs) multipartie à partir d'un échantillon de sang entier ayant au moins une population de globules rouges et des populations de WBC à l'intérieur, qui comprend les étapes suivantes :
(i) détecter et compter électroniquement au moins les populations de WBC de granulocytes, monocytes et lymphocytes en utilisant un signal à haute et basse fréquences;
(ii) éliminer ou déplacer de la contribution de la population de neutrophiles des populations de WBC, en mélangeant avec les microsphères échantillons comme éfini à la revendication 1;
(iii) détecter et compter électroniquement d'au moins les populations restantes de WBC de monocytes, de lymphocytes, d'éosinophiles et de basophiles; et
(iv) comparer les deux comptages, pour obtenir un comptage de la population de WBC de neutrophiles et de ce fait, au moins un différentiel de WBC de cinq parties.

3. Méthode selon la revendication 2, dans laquelle l'étape (i) est menée sur une première portion de l'échantillon, et les étapes (ii) et (iii) sont menées sur une seconde portion de l'échantillon.

4. Méthode d'analyse d'une population de WBC (globules blancs) dans un échantillon de sang entier comprenant une population de globules rouges et des populations de WBC, parmi lesquelles une population de WBC sélectionnée a au moins deux sous-séries, qui comprend les étapes suivantes :
éliminer ou déplacer au moins une contribution de sous-séries de populations de WBC dans la population de WBC sélectionnée en mélangeant avec les microsphères échantillons ayant, fixé à celles-ci, un anticorps monoclonal spécifique d'au moins une sous-série; et
détecter et analyser électroniquement la sous-série ainsi soustraite et la population de WBC en utilisant un signal à haute et basse fréquences, pour déterminer au moins une caractéristique de la population de WBC sélectionnée.

5. Méthode de détermination d'une population ou d'une sous-série de population de WBC (globules blancs) dans un échantillon de sang entier, qui comprend les étapes suivantes :
déplacer au moins une contribution aux caractéristiques de détection électronique d'une sous-série ou population de WBC par rapport à une autre population ou population de sous-série WBC, en mélangeant avec les microsphères échantillons ayant, fixé à celles-ci, un anticorps monoclonal spécifique d'au moins une population ou sous-série de WBC; et
détection électronique et comptage direct de la population déplacée en utilisant un signal à basse et haute fréquences pour obtenir la contribution à l'échantillon de la population ou sous-série déplacée.

6. Méthode d'analyse d'une population ou d'une sous-série de population de WBC (globules blancs) dans un échantillon de sang entier comprenant des populations de WBC incluant au moins une sous-série de population de WBC, qui comprend les étapes de:
détection électronique et comptage des populations de WBC et sous-séries de celle-ci en utilisant un signal de basse et haute fréquences, pour donner un premier comptage;
éliminer ou déplacer au moins l'une des populations de WBC ou une série de celle-ci en mélangeant avec les microsphères échantillons comme défini à la revendication 5;
détecter et compter électroniquement les populations de WBC restantes et/ou déplacées en utilisant un signal de haute et de basse fréquences pour donner un second comptage; et
comparer les premier et second comptages pour obtenir ou dériver l'une ou les deux des populations de WBC restantes et de la population ou population de sous-séries de WBC éliminées ou déplacées.

7. Méthode selon l'une quelconque des revendications précédentes, qui comprend l'étape supplémentaire antérieure de retirer la population de globules rouges de l'échantillon ou des portions de celle-ci, sans affecter de manière néfaste les qualités et/ou quantités pertinentes des populations de WBC.

8. Méthode selon l'une quelconque des revendications précédentes, dans laquelle la détection et le comptage ou l'analyse est celle ou celui du volume et de l'opacité des cellules.
